# EUROPEAN PATENT APPLICATION

(11) **EP 4 821 537 A1**
(43) Date of publication of application: **14.10.2026**
(21) Application number: 25760725.9
(22) Date of filing: 06.02.2025
(51) Int. Cl.: A61M 25/10, A61M 5/36

(54) **LIQUID PUMP, LIQUID FILLING SYSTEM, AND MEDICAL MICROCIRCULATION DEVICE**

(30) Priority: 27.02.2024 CN 202410217011
(71) Applicant: Shanghai Microport Rotapace Medtech Co., Ltd., Shanghai 201203 (CN)
(72) Inventor: LAN, Tianyu, Shanghai 201203 (CN); LIU, Chen, Shanghai 201203 (CN); YAO, Yingzhong, Shanghai 201203 (CN); ZHAO, Ruihui, Shanghai 201203 (CN); YUE, Bin, Shanghai 201203 (CN)
(74) Representative: Marks & Clerk LLP
(86) International application number: PCT/CN2025/075879
(87) International publication number: WO 2025/180181

(57) **Abstract**

A liquid pump (10), a liquid filling system and a medical microcirculation device are disclosed. The liquid pump (10) includes a pump body (100), an inflation/deflation mechanism (200) and a venting mechanism (300). The pump body (100) includes a liquid storage chamber (110), a venting chamber (120) and a transition chamber (130) joining the liquid storage chamber (110) to the venting chamber (120). The transition chamber (130) is disposed above, and communicates with, the liquid storage chamber (110). The pump body (100) also includes a connector (140) adapted for connection with a balloon catheter (30) and communicating with the transition chamber (130). The inflation/deflation mechanism (200) is disposed, at least in part, within the liquid storage chamber (110) so as to be able to reciprocate along an axis thereof to inflate and/or deflate a balloon. The venting mechanism (300) is disposed, at least in part, within the venting chamber (120) so as to be able to reciprocate along an axis thereof to bring the venting chamber (120) into communication with, or isolate it from, an external environment. This liquid pump (10) can address the problems of possible gas embolism caused by gas leakage and complex venting operations encountered in clinical practice.

## Description

### TECHNICAL FIELD

The present invention relates to the field of medical devices, and more particularly to a liquid pump, a liquid filling system and a medical microcirculation device.

### BACKGROUND

Percutaneous coronary intervention (PCI), which has been developed essentially to treat obstructive coronary disease, has been advancing rapidly since its debut thanks to the advantages of being minimally invasive, time-efficient, safe and highly effective. However, in some instances, coronary obstruction may cause ischemic damage to part of the myocardium. Such ischemia affects the coronary microcirculatory system most. Abnormalities in the microcirculatory system may cause hibernation, stunning, or even necrosis of a portion of the myocardium, which may in turn precipitate acute myocardial infarction. Regular PCI procedures cannot cope with insufficient perfusion in the microcirculatory system. Currently, this condition is usually treated by occluding blood flow through the coronary sinus to elevate vascular pressure, thereby forcing blood to flow back into the microcirculatory system to enhance its perfusion. This retrograde blood flow can also flush the microcirculatory system. One existing method for microcirculatory treatment is to use a medical device, which combines a pressurizer with a balloon catheter. A balloon of the balloon catheter is placed in the coronary sinus and repeatedly inflated and deflated to intermittently occlude the coronary sinus to provide the blood flow reversing and flushing actions described above.

However, existing medical microcirculation devices are associated with the following problems:
1. They are active devices intended for continuous use for a long period of time (typically, two hours), during which accidental leakage of an inflation medium may create a risk of irreversible harm. Moreover, the inflation medium is a gas (helium), which when leaked, may cause intravascular gas embolism, an emergency that, in severe cases, may prove fatal to the patient.
2. They feature a gas pump integrated with a main unit. Such integration means complicated maintenance, which has been recognized as one of the challenges they are faced with. This is because once either of the integrated components malfunctions, the repair will take much time and be expensive. Additionally, exchanging either of the components for a new one is indeed equivalent to purchasing a new device, ultimately placing additional strain on already limited medical resources. In particular, as a critical functional component, the pump has a relatively high probability of failure, and once it fails, the painful consequences discussed above will be entailed.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

It is an object of the present invention to provide a liquid pump, a liquid filling system and a medical microcirculation device, which not only allow a balloon to be inflated by liquid filling, thus circumventing the problem of possible gas leakage arising from the use of an inflating gas in the current clinical practice, which may cause gas embolism that threatens the patient's life, but also overcome the problem of complex venting operations.

To this end, the present invention provides a liquid pump for use in a medical microcirculation device, which includes a pump body, an inflation/deflation mechanism and a venting mechanism. The pump body includes a liquid storage chamber, a venting chamber and a transition chamber joining the liquid storage chamber to the venting chamber. The transition chamber is disposed above, and communicates with, the liquid storage chamber. The pump body also includes a connector adapted for connection with a balloon catheter and communicating with the transition chamber. The inflation/deflation mechanism is disposed, at least in part, within the liquid storage chamber so as to be able to reciprocate along an axis thereof to inflate and/or deflate a balloon. The venting mechanism is disposed, at least in part, within the venting chamber so as to be able to reciprocate along an axis thereof to bring the venting chamber into communication with, or isolate it from, an external environment.

Optionally, the inflation/deflation mechanism may include a transmission assembly and a piston, which are coupled to each other, the piston including a piston rod and a piston head, which are coupled to each other, the piston head disposed within the liquid storage chamber, the piston extending, at least in part, outside the liquid storage chamber and drivingly coupled to the transmission assembly, the transmission assembly coupled to a power device and configured to be driven by the power device to cause the piston rod and the piston head to reciprocate along the axis of the liquid storage chamber.

Optionally, the transmission assembly may include a coupling and a gear coaxially coupled to the coupling, wherein the piston rod is drivingly coupled to the gear, and the coupling is coupled to the power device.

Optionally, there may be a plurality of teeth on one side of the piston rod, which is configured to mesh with the gear.

Optionally, the piston rod may be eccentrically coupled to the gear via a joint member.

Optionally, the liquid storage chamber may include a tapered portion at an end thereof proximal to the transition chamber, an inner diameter of the tapered portion at its end proximal to the transition chamber less than an inner diameter of the tapered portion at its end distal from the transition chamber.

Optionally, the venting mechanism may include a press button, an air valve and a venting rod, a top end of the venting rod coupled to the press button, the air valve movably disposed over the venting rod, wherein: the pump body further includes a mounting chamber joined to the venting chamber and communicating with the external environment; top ends of the press button and the venting rod are both disposed within the mounting chamber; the press button is able to reciprocate along an axis of the mounting chamber; a bottom end of the venting rod is disposed inside the venting chamber; the air valve is located at the joint member of the mounting chamber and the venting chamber; when the press button is at rest, the bottom end of the venting rod seals a bottom end of the air valve, thereby isolating the venting chamber from the mounting chamber; when the press button is pressed, the venting rod is caused to move toward the transition chamber relative to the air valve, thereby bringing the venting chamber into communication with the mounting chamber.

Optionally, the liquid pump may further include a locking member movably coupled to a wall of the mounting chamber below the press button and above the air valve, the locking member extending, at least in part, outside the mounting chamber, the locking member configured to, when there is no need for venting, abut against the press button, thereby placing the venting mechanism into a locked state.

Optionally, the venting rod may include a rod body and a sealing head, which are coupled to each other, the sealing head having a diameter greater than a diameter of the rod body, a top end of the rod body coupled to the press button, wherein the air valve defines a first bore and a second bore underlying and communicating with the first bore, the second bore having a greater inner diameter than the first bore and configured for insertion of the sealing head therein with an interference fit, the first bore configured for insertion of the rod body therethrough.

Optionally, the venting mechanism may further include a resilient member coupled to the press button or the venting rod, the resilient member configured to, when an external force exerted on the press button is removed, bias the venting rod and the press button back to their own rest positions.

Optionally, the venting mechanism may further include an air-valve cap disposed within the mounting chamber on the venting rod above the air valve, the air-valve cap having a diameter greater than the inner diameter of the first bore, wherein when the press button is at rest, the air-valve cap is spaced from the air valve at a distance greater than a depth of the second bore.

Optionally, the venting mechanism may further include a ball valve coupled to the bottom end of the venting rod, and the venting chamber may define, at an end thereof proximal to the transition chamber, an air inlet port, in which the ball valve is able to be fitted.

Optionally, the mounting chamber may be provided therein with a stop member between the press button and the air valve.

To the above end, the present invention also provides a liquid filling system including a main unit and a liquid pump as defined above. The main unit is detachably coupled to the liquid pump.

To the above end, the present invention also provides a medical microcirculation device including a balloon catheter and a liquid filling system as defined above.

The liquid pump, the liquid filling system and the medical microcirculation device, as discussed above, offer the following benefits over the prior art:
The liquid pump includes a pump body, an inflation/deflation mechanism and a venting mechanism. The pump body includes a liquid storage chamber, a venting chamber and a transition chamber joining the liquid storage chamber to the venting chamber. The transition chamber is disposed above, and communicates with, the liquid storage chamber. The pump body also includes a connector adapted for connection with a balloon catheter and communicating with the transition chamber. The inflation/deflation mechanism is disposed, at least in part, within the liquid storage chamber so as to be able to reciprocate along an axis thereof to inflate and/or deflate a balloon. The venting mechanism is disposed, at least in part, within the venting chamber so as to be able to reciprocate along an axis thereof to bring the venting chamber into communication with, or isolate it from, an external environment. With this arrangement, the liquid storage chamber and the inflation/deflation mechanism in the liquid pump allow for balloon inflation by liquid filling, reducing the risk of gas embolism caused by gas leakage during use. Moreover, the transition chamber, the venting chamber and the venting mechanism in the liquid pump overcome the problem of complex venting operations, while providing satisfactory venting during liquid filling. Further, the liquid pump can be provided as a separate functional component or consumable for one-time use, allowing for easier and simpler maintenance of the liquid pump.

The liquid filling system and the medical microcirculation device both incorporate the liquid pump and, therefore, offer at least all the benefits of the liquid pump. These benefits are not repeated herein, and reference is made to the above description in connection with the liquid pump for more details of them. Since the liquid pump contains a liquid, which can be communicated into a balloon, in an extreme case, if the balloon ruptures within a human blood vessel, the liquid would leak from the liquid pump and come into direct contact with blood in the human body. Therefore, it would be necessary to sterilize and disinfect the liquid pump each time before clinical use. According to the present invention, since the liquid pump is detachably coupled to the main unit in both the liquid filling system and the medical microcirculation device, the liquid pump could be sterilized by the manufacturer and then provided in a sterile form. This allows for direct clinical use of a fresh, sterile liquid pump every time by a user, and coupled with the fact that sterile saline or a similar sterile liquid that is readily available in an operating room could be used as the liquid in the liquid pump, dispenses with repeatedly sterilization and disinfection of the liquid filling system and medical microcirculation device by hospital personnel, addressing the problem of sterilization and disinfection of the liquid pump and the main unit that would be otherwise necessary every time before clinical use if the liquid pump is integrated with the main unit. Further, the design with the liquid pump being detachably coupled to the main unit enables easy and convenient maintenance of the liquid pump and, in the event of the liquid pump being damaged or otherwise rendered inoperable, allows it to be readily exchanged for a new one that could be quickly assembled on the main unit.

### BRIEF DESCRIPTION OF THE DRAWINGS

Fig. 1 is a schematic diagram showing the overall structure of a liquid pump, according to an embodiment of the present invention.
Fig. 2 is a schematic diagram illustrating how a transmission assembly is coupled to a power device, in accordance with an embodiment of the present invention.
Fig. 3 is a schematic diagram illustrating how a transmission assembly is coupled to a piston, in accordance with an alternative embodiment of the present invention.
Fig. 4 is a schematic illustration of a venting mechanism in a locked state, according to an embodiment of the present invention.
Fig. 5 is a schematic illustration of a venting mechanism in an unlocked state, according to an embodiment of the present invention.
Fig. 6 is a schematic illustration of a venting mechanism in a configuration, where air can be vented out, according to an embodiment of the present invention.
Fig. 7 is a schematic illustration of a venting mechanism in a configuration, where an air-valve cap seals a top end of an air valve, according to an embodiment of the present invention.
Fig. 8 is a schematic illustration of a venting mechanism in a configuration, where a venting rod again seals a bottom end of an air valve, according to an embodiment of the present invention.
Fig. 9 is a schematic diagram illustrating how a venting rod is coupled to an air valve, in accordance with an embodiment of the present invention.
Fig. 10 shows a cross-sectional view of an air valve, according to an embodiment of the present invention.
Fig. 11 shows a schematic partial view of a press button at rest in a venting mechanism, according to an embodiment of the present invention.
Fig. 12 is a schematic diagram showing a press button at rest in a venting mechanism, according to an alternative embodiment of the present invention.
Fig. 13 is a schematic illustration of a venting mechanism in a configuration, where air can be vented out, according to an alternative embodiment of the present invention.
Fig. 14 is a schematic diagram showing the overall structure of a liquid pump, according to an alternative embodiment of the present invention.
Fig. 15 shows a schematic block diagram of a liquid filling system, according to an embodiment of the present invention.
Fig. 16 shows a schematic block diagram of a medical microcirculation device, according to an embodiment of the present invention.

### List of Reference Numerals

100 pump body; 110 liquid storage chamber; 111 tapered portion; 120 venting chamber; 121 air inlet port; 130 transition chamber; 140 connector; 150 mounting chamber; 151 stop member; 160 locking member;
200 inflation/deflation mechanism; 210 transmission assembly; 211 coupling; 212 gear; 220 piston; 221 piston rod; 2211 tooth; 222 piston head; 230 joint member;
300 venting mechanism; 310 press button; 320 air valve; 321 first bore; 322 second bore; 330 venting rod; 331 rod body; 332 sealing head; 340 ball valve; 350 resilient member; 360 air-valve cap;
10 liquid pump;
20 main unit; 21 power device;
30 balloon catheter.

### DETAILED DESCRIPTION

A liquid pump, liquid filling system and medical microcirculation device proposed herein will be described in greater detail below with reference to the accompanying drawings, which illustrate specific embodiments of the present invention. From the following description, the advantages and features of the present invention will become more apparent. Note that the figures are heavily simplified and not necessarily drawn to scale. They are presented only to assist in facilitating clear illustration of the embodiments disclosed herein. In order that the objects, features and advantages of the present invention can be more apparent and readily understood, reference is to be made to the accompanying drawings. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein. The specific design features of the invention as disclosed herein, including, for example, specific dimensions, orientations, locations and shapes, will be determined in part by the particular intended application and use environment. Additionally, in the embodiments described below, like reference numerals may be sometimes used to refer to the same or functionally similar elements throughout different figures, while description thereof may not be repeated. In this specification, similar reference numerals and letters refer to similar items in the figures, and thus once an item is defined in one figure, it may not be discussed for following figures. Further, if a method is described herein as including a series of steps, the order of these steps as presented herein is not necessarily the only order in which they can be performed, and some of the stated steps may be omitted and/or other steps not described herein may be added to the method.

It is to be noted that relational terms such as "first," "second," and the like may be used herein solely to distinguish one entity or action from another entity or action, without necessarily requiring or implying any actual such relationship or order between such entities or actions, or denoting or implying relative importance of them, or implicitly indicating the number of them. Moreover, the terms "comprise", "include" and any variations thereof are intended to cover a non-exclusive inclusion, such that a process, method, article, or apparatus that comprises a list of elements is not necessarily limited to those elements, but may include other elements not expressly listed or inherent to such process, method, article, or apparatus. An element preceded by "comprises... a" does not, without more constraints, preclude the existence of additional identical elements in the process, method, article, or apparatus that comprises the element. As used herein, the singular forms "a", "an" and "the" include plural referents, and the term "or" is generally employed in the sense of "and/or", "a number of" of "at least one", "at least two" of "two or more", and "plurality" of "at least two".

It should be understood that the terms such as "central", "longitudinal", "transverse", "length", "width", "thickness", "upper", "lower", "front", "rear", "left", "right", "vertical", "horizontal", "top", "bottom", "inner", "outer", "axial", "radial", "circumferential", etc. may be used herein to describe directional or positional relationships based on the orientations shown in the figures. They are intended merely to facilitate and simplify the explanation of the present invention and do not indicate or imply that the stated components or elements have to assume, or be constructed or operated in, particular orientations. Therefore, they are not to be construed as limiting the application. As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context. Further, as used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature .When a first feature is referred to as being "under", "below" or "at bottom of" a second feature, it may be right or obliquely under, below or at bottom of the second feature, or simply located at a lower level than the second feature .

It is a principal object of the present invention to provide a liquid pump, a liquid filling system and a medical microcirculation device, which not only allow a balloon to be inflated by liquid filling, thus circumventing the problem of possible gas leakage arising from the use of an inflating gas in the current clinical practice, which may cause gas embolism that threatens the patient's life, but also overcome the problem of complex venting operations. The invention can also address the problem of difficult maintenance associated with devices incorporating integrated components. It should be noted and will be appreciated by those skilled in the art that, in addition to balloon pumping in the coronary sinus, the medical microcirculation device can also be used in other applications, without departing from the scope of the present invention.

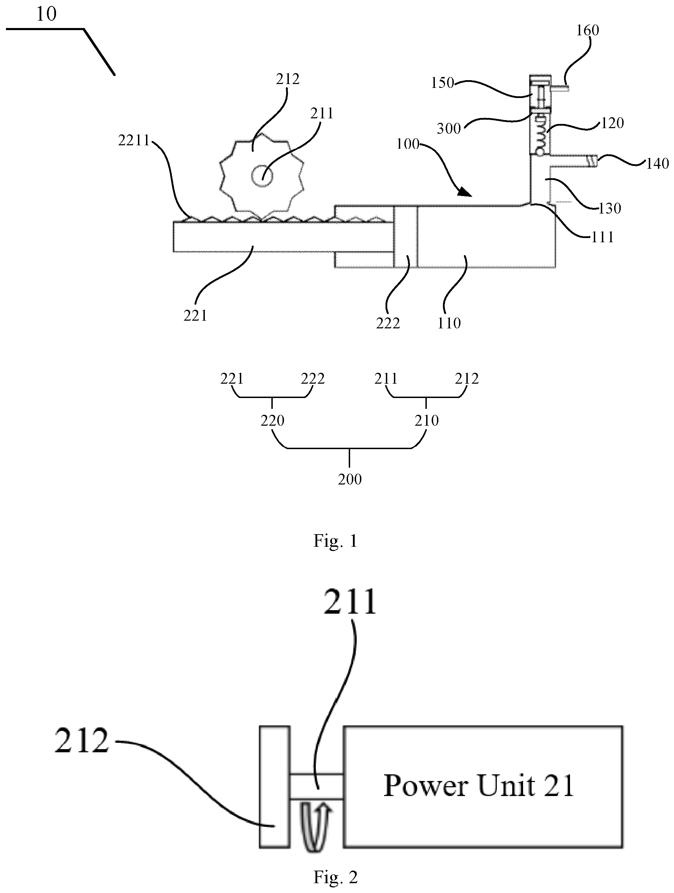

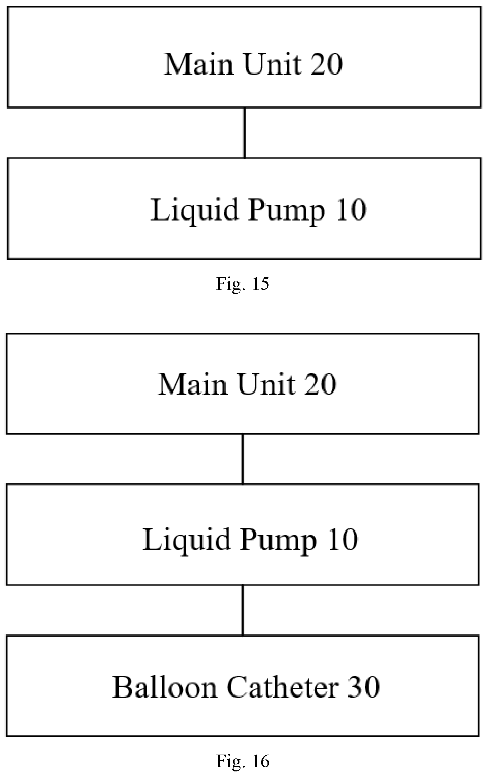

The above object is attained by a liquid pump proposed herein, which is intended for use in a medical microcirculation device. Reference is now made to Fig. 1, a schematic diagram showing the overall structure of a liquid pump according to an embodiment of the present invention. As shown in Fig. 1, the liquid pump 10, as disclosed herein, includes a pump body 100, an inflation/deflation mechanism 200 and a venting mechanism 300. The pump body 100 has a liquid storage chamber 110, a venting chamber 120 and a transition chamber 130 joining the liquid storage chamber 110 to the venting chamber 120. The transition chamber 130 is disposed above, and communicates with, the liquid storage chamber 110. The pump body 100 also has a connector 140 adapted for connection with a balloon catheter 30 (see Fig. 16). The connector 140 communicates with the transition chamber 130. At least a portion of the inflation/deflation mechanism 200 is disposed within the liquid storage chamber 110 such as to be able to reciprocate along an axis thereof to enable balloon inflation and/or deflation. At least a portion of the venting mechanism 300 is disposed within the venting chamber 120 such as to be able to reciprocate along an axis thereof to bring the venting chamber 120 into communication with, or isolate it from, the external environment.

The liquid storage chamber 110 and the inflation/deflation mechanism 200 in the liquid pump 10, as disclosed herein, allow for balloon inflation by liquid filling, reducing the risk of gas embolism caused by gas leakage during use. In addition, the transition chamber 130, the venting chamber 120 and the venting mechanism 300 in the liquid pump 10, as disclosed herein, overcome the problem of complex venting operations, while providing satisfactory venting during liquid filling. Further, the liquid pump 10, as disclosed herein, can be provided as a separate functional component or consumable for one-time use, allowing for easier and simpler maintenance of the liquid pump 10.

It should be noted and will be appreciated by those skilled in the art that, prior to balloon inflation, the pump body 100 may be connected, via the connector 140, thereof to a liquid reservoir that contains an inflating liquid, and the inflating liquid may be sucked by the inflation/deflation mechanism 200 from the liquid reservoir into the liquid storage chamber 110 until the liquid storage chamber 110 is fully filled with the inflating liquid. Subsequently, the connector 140 of the pump body 100 may be connected to a balloon, and before the balloon is cyclically inflated, air in the pump body 100 may be vented through the venting mechanism 30, without undesirably escaping into the balloon. Afterwards, the inflating liquid may be supplied by the inflation/deflation mechanism 200 from the liquid storage chamber 110 into the balloon to cause its inflation. Also, it should be noted and will be appreciated by those skilled in the art that the venting of air can be accomplished simply through bringing the venting chamber 120 into communication with the external environment by the venting mechanism 300. Further, it should be noted and will be appreciated by those skilled in the art that the connector 140 may be, but is not limited to being, a Luer connector. Alternatively, it may also be a special connector for dedicated use with a certain type of balloon catheter.

Continued reference is made to Fig. 1. As shown in Fig. 1, in some exemplary embodiments, the inflation/deflation mechanism 200 includes a transmission assembly 210 and a piston 220, which are coupled to each other. The piston 220 includes a piston rod 221 and a piston head 222 coupled to the piston rod 221. The piston head 222 is disposed within the liquid storage chamber 110, and the piston rod 221 extends, at least in part, outside the liquid storage chamber 110 and is drivingly coupled to the transmission assembly 210. The transmission assembly 210 is coupled to a power device 21 (see Fig. 2) and configured to be driven by the power device 21 to cause the piston rod 221 and the piston head 222 to reciprocate along the axis of the liquid storage chamber 110. As a result of the piston rod 221 and the piston head 222 being caused to reciprocate along the axis of the liquid storage chamber 110 by the transmission assembly 210 under the drive of the power device 21, the inflating liquid is transferred from the liquid storage chamber 110 into the balloon to cause its inflation, or sucked out from the balloon to cause its deflation. Specifically, as viewed in the orientation of Fig. 1, when the piston rod 221 and the piston head 222 are driven by the power device 21 and the transmission assembly 210 to move to the right, the inflating liquid is transferred from the liquid storage chamber 110 into the balloon, causing its inflation. On the contrary, when the piston rod 221 and the piston head 222 are moved to the left under the drive of the power device 21 and the transmission assembly 210, the liquid is drawn out from the balloon, causing its deflation.

Reference is made again to Fig. 1, and also to Fig. 2, a schematic diagram illustrating how the transmission assembly 210 is coupled to the power device 21 according to an embodiment of the present invention. As shown in Figs. 1 and 2, in some exemplary embodiments, the transmission assembly 210 includes a coupling 211 and a gear 212 coaxially coupled to the coupling 211. The piston rod 221 is drivingly coupled to the gear 212, and the coupling 211 is coupled to the power device 21. This design of the transmission assembly 210 with the coupling 211 and the gear 212 coupled to the coupling 211 not only allows the liquid pump 10, as disclosed herein, to be simpler in structure, but also facilitates its coupling with the power device 21 via the coupling 211. Specifically, the power device 21 can drive rotation of the gear 212, which in turn cause the piston rod 221 that is drivingly coupled to the gear 212, and hence the piston head 222, to move linearly. It should be noted and will be appreciated by those skilled in the art that the power device 21 that is able to drive rotation of the gear 212 may be a rotary motor, or a rotary cylinder.

Continued reference is made to Fig. 1. As shown in Fig. 1, in some exemplary embodiments, there is a plurality of teeth 2211 on one side of the piston rod 221, which is configured to mesh with the gear 212. With this arrangement, when actuated to rotate by the power device 21, the gear 212 will drive the piston rod 221 that it meshes with, and hence the piston head 222, to linearly move along the axis of the liquid storage chamber 110.

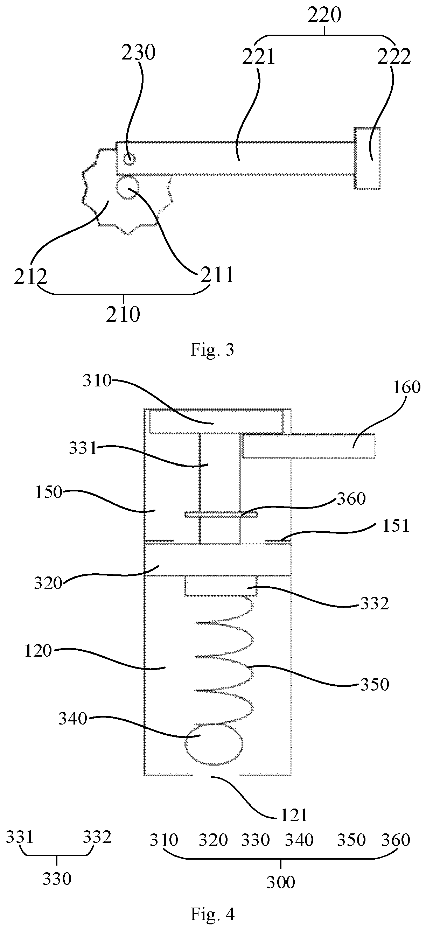

Additional reference is made to Fig. 3, a schematic diagram showing how the transmission assembly 210 is coupled to the piston 220, in accordance with another embodiment of the present invention. As shown in Fig. 3, in some other exemplary embodiments, the piston rod 221 is eccentrically coupled to the gear 212 with a joint member 230. With this arrangement, the power device 21 can drive rotation of the gear 212, which in turn causes the piston rod 221 that is eccentrically coupled to the gear 212, and hence the piston head 222, to linearly move along the axis of the liquid storage chamber 110.

Specifically, the joint member 230 may be eccentrically affixed to the gear 212 (i.e., at a location offset from the center of the circular gear 212) at one end and articulated to the piston rod 221 at the other end, with the piston rod 221 being in turn articulated to the piston head 222. With this arrangement, the gear 212, when rotated, drives the joint member 230 to rotate in synchronization therewith about an axis of the gear 212, and the rotating joint member 230 in turn cause deflections of the piston rod 221. Under the action of such deflections, the piston head 222 linearly moves along the axis of the liquid storage chamber 110.

It should be noted and will be appreciated by those skilled in the art that, in some alternative embodiments, the transmission assembly 210 may be a lead screw or other transmission mechanism. More details of the lead screw serving as a transmission mechanism, reference is made to literature on related techniques known to those skilled in the art, and further description thereof is omitted herein. Also, it should be noted and will be appreciated by those skilled in the art that, in some alternative embodiments, the power device 21 may be a linear motor. In these embodiments, the transmission assembly 210 may include only the coupling 211, which is instead coupled to the piston rod 221 at one end and to a mover in the linear motor at the other end.

Reference is again made to Fig. 1. As shown in Fig. 1, in some exemplary embodiments, the liquid storage chamber 110 has a tapered portion 111 at its end proximal to the transition chamber 130. An inner diameter of the tapered portion 111 at its end proximal to the transition chamber 130 is less than an inner diameter of the tapered portion 111 at its end distal from the transition chamber 130 (i.e., the tapered portion 111 has an inner diameter gradually increasing from the top downwards). With this arrangement, the tapered portion 111 disposed at the end of the liquid storage chamber 110 proximal to the transition chamber 130, which is narrower at the top and wider at the bottom, ensures that air, if present there, flows upwards. This effectively prevents air from being trapped within the liquid storage chamber 110.

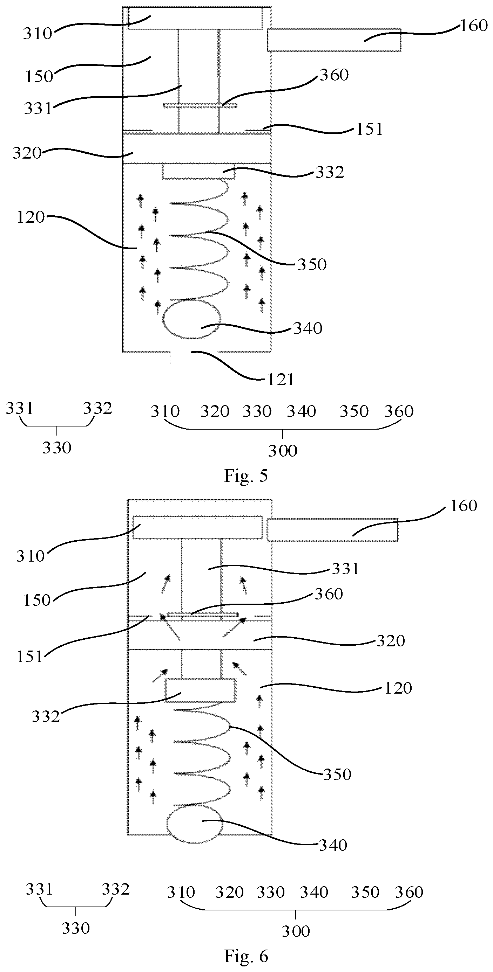

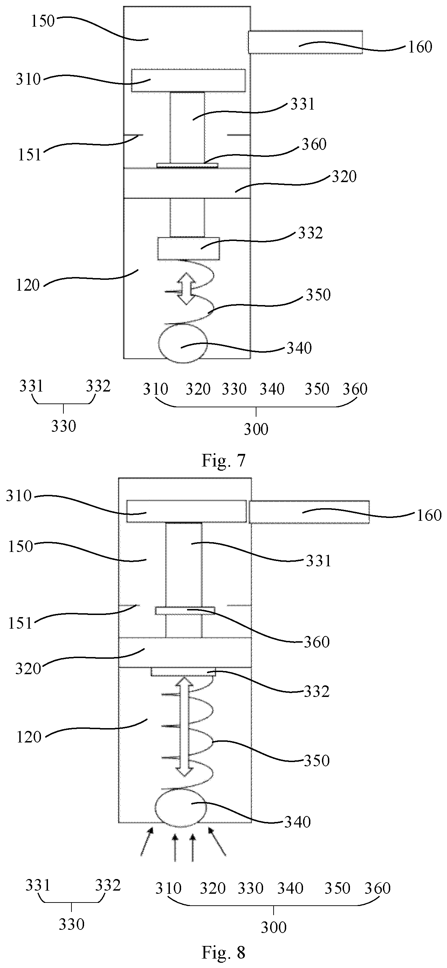

Reference is additionally made to Figs. 4 to 8. Fig. 4 is a schematic illustration of the venting mechanism 300 in a locked state according to an embodiment of the present invention. Fig. 5 is a schematic illustration of the venting mechanism 300 in an unlocked state according to an embodiment of the present invention. Fig. 6 is a schematic illustration of the venting mechanism 300 in a configuration, where air can be vented out, according to an embodiment of the present invention. Fig. 7 is a schematic illustration of the venting mechanism 300 in a configuration, where an air-valve cap 360 seals a top end of an air valve 320, according to an embodiment of the present invention. Fig. 8 is a schematic illustration of the venting mechanism 300 in a configuration, where a venting rod 330 again seals a bottom end of the air valve 320, according to an embodiment of the present invention. As shown in Figs. 4 to 8, in some exemplary embodiments, the venting mechanism 300 includes a press button 310, an air valve 320, a venting rod 330 and a ball valve 340. A top end of the venting rod 330 is coupled to the press button 310, and a bottom end thereof is couple to the ball valve 340. The air valve 320 is disposed on the venting rod 330. The venting chamber 120 defines, at a proximal end thereof to the transition chamber 130, an air inlet port 121, in which the ball valve 340 can be snugly fitted. The pump body 100 also has a mounting chamber 150, which is joined to the venting chamber 120 and communicates with the external environment. Top ends of the press button 310 and the venting rod 330 are both disposed within the mounting chamber 150, while the bottom end of the venting rod 330 and the ball valve 340 are both disposed inside the venting chamber 120. The air valve 320 is provided at the joint of the mounting chamber 150 and the venting chamber 120, with the bottom end of the venting rod 330 sealing a bottom end of the air valve 320 and isolating the venting chamber 120 from the mounting chamber 150. The press button 310 is able to reciprocate along an axis of the mounting chamber 150 to cause the venting rod 330 to reciprocate relative to the air valve 320 along an axis thereof. When the venting rod 330 is moved along its axis towards the air inlet port 121, its bottom end will move away from the air valve 320 and drive the ball valve 340 to seal the air inlet port 121. Conversely, when the venting rod 330 moves along its axis away from the air inlet port 121, its bottom end will again seal the bottom end of the air valve 320 and bring the ball valve 340 away from the air inlet port 121. This design of the venting mechanism 300 with the press button 310, the air valve 320, the venting rod 330 and the ball valve 340 not only ensures that air can be successfully vented through the venting mechanism 300, but also allows the liquid pump 10, as disclosed herein, to be remarkably simpler in structure, contributing to a compact design thereof. Moreover, the design of the venting mechanism 300 with the press button 310, the air valve 320, the venting rod 330 and the ball valve 340 allows for ease of operation - if necessary, an operator can manually operate the venting mechanism 300 to vent air to the external environment. It should be noted that, the single-headed arrows in Figs. 5, 6 and 8 indicate directions of air flow, while the double-headed arrows in Figs. 7 and 8 indicate directions of biasing of a resilient member 350.

Specifically, as shown in Fig. 4, when the press button 310 is at rest, the bottom end of the venting rod 330 seals the bottom end of the air valve 320, thereby isolating the venting chamber 120 from the mounting chamber 150 (i.e., blocking the venting chamber 120 from communication with the mounting chamber 150), and hence from the external environment, and disallowing release of air into the external environment. Meanwhile, when the press button 310 is at rest, the ball valve 340 does not seal the air inlet port 121, and the venting chamber 120 is in communication with the transition chamber 130, allowing entry of any gas or liquid into the venting chamber 120. As shown in Fig. 6, in order to allow for air venting, the press button 310 may be pressed down, causing the venting rod 330 to move, together with the ball valve 340, toward the air inlet port 121, until the ball valve 340 is fitted into and seals the air inlet port 121. In this process, the bottom end of the venting rod 330 increasingly moves away from the air valve 320, leaving the air valve 320 in an open state, where air present in the venting chamber 120 can flow into the mounting chamber 150 through the air valve 320, and further into the outside world from the mounting chamber 150. As shown in Fig. 8, when the venting rod 330 moves away from the air inlet port 121, the ball valve 340 will be dislodged from the air inlet port 121, leaving the air inlet port 121 in an open state. Upon the bottom end of the venting rod 330 coming into contact with the bottom end of the air valve 320, it seals the air valve 320, isolating the venting chamber 120 from the mounting chamber 150 (i.e., blocking the venting chamber 120 from communication with the mounting chamber 150), and hence from the external environment, and disallowing release of air into the external environment. These venting operations may be repeated until air is completely expelled.

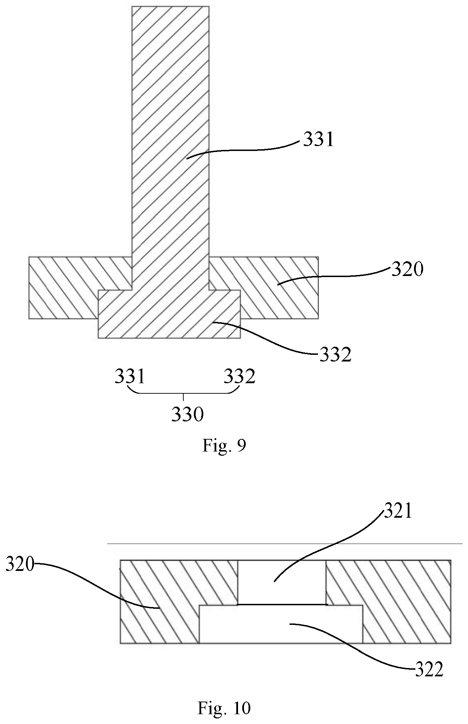

Reference is made again to Figs. 4 to 8, and also to Fig. 9, a schematic diagram illustrating how the venting rod 330 is coupled to the air valve 320 in accordance with an embodiment of the present invention. As shown in Figs. 4 to 9, in some exemplary embodiments, the venting rod 330 includes a rod body 331 and a sealing head 332 coupled to the rod body 331. The sealing head 332 has a greater diameter than the rod body 331. A top end of the rod body 331 is coupled to the press button 310, and a bottom end of the sealing head 332 is coupled to the ball valve 340. Reference is additionally made to Fig. 10, which shows a cross-sectional view of the air valve 320 according to an embodiment of the present invention. As shown in Fig. 10, the air valve 320 defines a first bore 321 and a second bore 322, which communicate with each other. The second bore 322 underlies and has a greater inner diameter than the first bore 321. The second bore 322 is configured for insertion of the sealing head 332 therein with an interference fit, and the first bore 321 is configured for insertion of the rod body 331 therethrough. The design of the venting rod 330 with the rod body 331 and the sealing head 332 coupled to the rod body 331 and the design of the air valve 320 with the first bore 321 complementary to the rod body 331 and the second bore 322 complementary to the sealing head 332 not only ensure that the bottom end of the venting rod 330 (i.e., the sealing head 332) can successfully seal the air valve 320 but also always allow the venting rod 330 to reciprocate along its axis. Additionally, these designs facilitate return of the venting mechanism 300 to its rest configuration. In addition, since the second bore 322 has a greater inner diameter than the first bore 321, it is ensured that the bottom end of the venting rod 330 (i.e., the sealing head 332), when received in the second bore 322, can successfully seal the air valve 320.

It should be noted and will be appreciated by those skilled in the art that the rod body 331 should have a diameter that is slightly less than the inner diameter of the first bore 321. With this arrangement, the sealing head 332 can be moved away from (contact with) the air valve 320, leaving the air valve 320 in an open state, where the venting chamber 120 communicates with the mounting chamber 150, allowing release of air from the venting chamber 120.

Continued reference is made to Figs. 4 to 8. As shown in Figs. 4 to 8, in some exemplary embodiments, the venting mechanism 300 further includes a resilient member 350. A top end of the resilient member 350 is coupled to the bottom end of the venting rod 330, and a bottom end thereof is coupled to the ball valve 340. The resilient member 350 is configured to, when an external force exerted on the press button 310 is removed, drive the venting rod 330, and hence the ball valve 340 and the press button 310, to return to their corresponding rest positions. With this arrangement, once an operator releases the press button 310 (i.e., an external force exerted on the press button 310 is removed), the resilient member 350 in the venting mechanism 300 will bias the venting rod 330, and hence the ball valve 340 and the press button 310, successfully back to their corresponding rest positions, resulting in greater ease of operation. It should be noted and will be appreciated by those skilled in the art that the resilient member 350 may be, but is not limited to being, implemented as a spring, an elastic tab or the like.

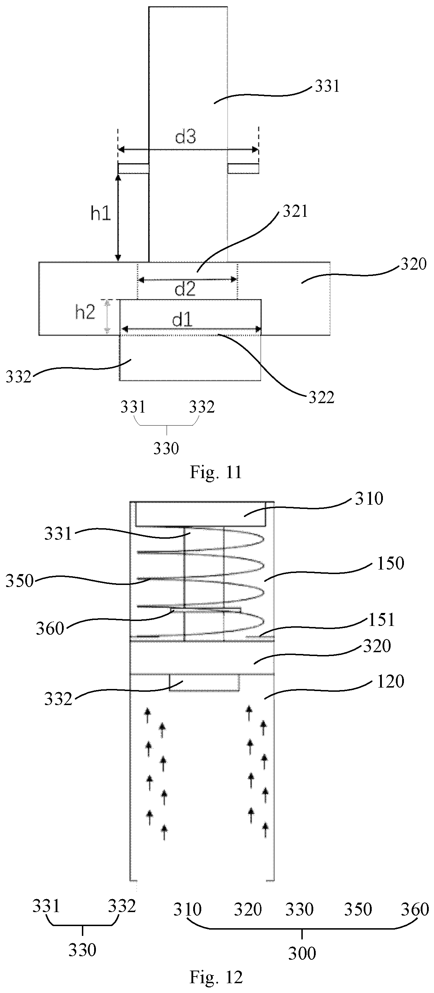

Continued reference is made to Figs. 4 to 8. As shown in Figs. 4 to 8, in some exemplary embodiments, the venting mechanism 300 further includes an air-valve cap 360 disposed in the mounting chamber 150 on the venting rod 330 above the air valve 320. Additional reference is made to Fig. 11, which shows a schematic partial view of a press button at rest in a venting mechanism according to an embodiment of the present invention. As shown in Fig. 11, the air-valve cap 360 has a diameter d3 greater than the inner diameter d2 of the first bore 321. When the press button 310 is at rest, the air-valve cap 360 is spaced from the air valve 320 at a distance h1 greater than a depth h2 of the second bore 322. Since the diameter d3 of the air-valve cap 360 is greater than the inner diameter d2 of the first bore 321, it can move with the venting rod 330 toward the air inlet port 121 (i.e., toward the transition chamber 130) to seal a top end of the air valve 320 and in turn cause the air valve 320 to move toward the air inlet port 121. Thus, the air-valve cap 360, after coming into contact with the top end of the air valve 320, can further move downwards with the venting rod 330 to cause the air valve 320 to in turn move downwards with it against the resilient member. Moreover, since the distance h1 between the air-valve cap 36 and the air valve 320 is greater than the depth h2 of the second bore 322 when the press button 310 is at rest, it is ensured that before the air-valve cap 360 seals the top end of the air valve 320, the sealing head 332 of the venting rod 330 has been separated from the air valve 320, allowing for release of air. It should be noted and will be appreciated by those skilled in the art that d1 in Fig. 11 denotes the inner diameter of the second bore 322.

Specifically, as shown in Fig. 8, once the press button 310 is released, the venting rod 330 is biased by the resilient member 350 to move upwards, causing the air-valve cap 360 to move away from the air valve 320. At this point, since the air valve 320 is spaced from the sealing head 332 of the venting rod 330 at a certain distance, the air valve 320 is not closed immediately. Instead, the air valve 320 is closed at a later time when the sealing head 332 comes into contact with it. As a result of the air valve 320 being closed, the venting chamber 120 is isolated from the external environment. Moreover, at this point, as the resilient member 350 still biases the venting rod 330, the latter is further moved upwards, causing movement of the air valve 320, the ball valve 340, the air-valve cap 360 and the press button 310 towards their rest positions. This creates a negative pressure within the venting chamber 120, which in turn creates an effect of sucking the liquid and air away from the balloon.

Continued reference is made to Figs. 1 and 4 to 8. As shown in Figs. 1 and 4 to 8, in some exemplary embodiments, the liquid pump 10 further includes a locking member 160 movably attached to a wall of the mounting chamber 150 below the press button 310 and above the air valve 320. At least part of the locking member 160 extends outside the mounting chamber 150. The locking member 160 configured to, when there is no need for venting, maintain the venting mechanism 300 in a locked state by abutting against the press button 310. With this arrangement, when there is no need for venting, the locking member 160 may be moved into abutment with the bottom of the press button 310 to prevent accidental triggering of the press button 310 during balloon inflation, which may create a negative pressure in the liquid storage chamber 110. Such a negative pressure can have an adverse impact on the venting system because it may disallow complete balloon inflation. Additionally, the locking member 160 provides support for the press button 310, ensuring that the press button 310 and the ball valve 340 reliably remain at their rest positions in the absence of any external force. It should be noted that, as shown in Fig. 5, in order to allow for venting, the locking member 160 may be displaced outwardly until it is separate from the press button 310, before the press button 310 can be pressed down. After air is completely expelled, prior to cyclic balloon inflation, the locking member 160 may be again moved into abutment with the bottom of the press button 310, bringing the venting mechanism 300 back into a locked state.

Continued reference is made to Figs. 4 to 8. As shown in Figs. 4 to 8, in some exemplary embodiments, the mounting chamber 150 is further provided therein with a stop member 151 between the press button 310 and the air valve 320. With this arrangement, the stop member 151 provided in the mounting chamber 150 can stop the air valve 320 from undesired further movement, effectively ensuring that the air valve 320 can return to its rest position under the action of a biasing force of the resilient member 350.

It should be noted and will be appreciated by those skilled in the art that the stop member 151 should be positioned so as to avoid interfering with the air-valve cap 360 or the venting rod 330, thus ensuring that the air-valve cap 360 and the venting rod 330 are free to move vertically up and down. Also, it should be noted and will be appreciated by those skilled in the art that the present invention is not limited to any particular structure of the stop member 151. The stop member 151 may be, but is not limited to being, implemented as a disc, as long as it is able to stop the air valve 320 from further movement while not interfering with vertical movement of the air-valve cap 360 or the venting rod 330.

In some exemplary embodiments, the liquid pump 10 further includes a housing (not shown), in which the inflation/deflation mechanism 200 is enclosed, and which is provided thereon with a feature for snap engagement with a shell of the main unit 20, as described below. This allows for secure attachment of the liquid pump 10 to the main unit 20. It should be noted and will be appreciated by those skilled in the art that the portion of the transmission assembly 210 to be coupled to the power device 21 in the main unit 20 should protrude out of the housing.

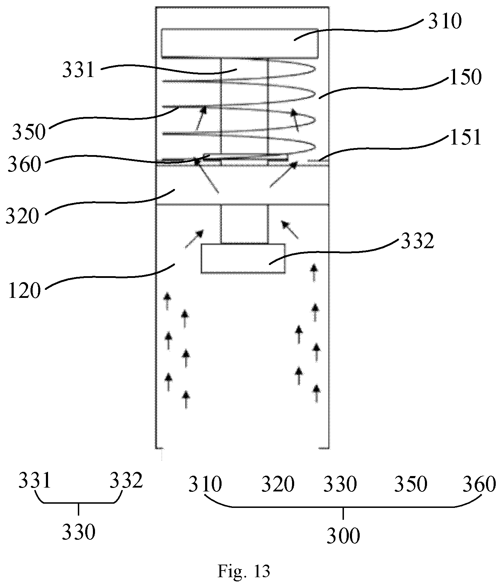

Reference is additionally made to Figs. 12 and 13. Fig. 12 is a schematic diagram showing a press button at rest in a venting mechanism, according to an alternative embodiment of the present invention. Fig. 13 is a schematic illustration of a venting mechanism in a configuration, where air can be vented out, according to an alternative embodiment of the present invention. As shown in Figs. 12 and 13, in other embodiments than the preceding ones shown in Figs. 5 to 8, the ball valve 340 may be omitted from the venting mechanism 300, with the resilient member 350 being coupled to the bottom end of the press button 310 at its top end and to the stop member 151 at its bottom end. This enables the venting mechanism 300 to have an even simpler structure, when compared with the preceding embodiments.

It should be noted and will be appreciated by those skilled in the art that, in these alternative embodiments, supported by the resilient member 350, the press button 310 is able to stay stationary at the rest position if there is no external force applied thereto. For this reason, the locking member 160 may be omitted in these embodiments. Of course, in order to prevent accidental triggering, as with the cases shown in Figs. 5 to 8, the venting mechanism 300 in these embodiments may also be provided with a locking member 160 movably coupled to the wall of the mounting chamber 150. Also, it should be noted and will be appreciated by those skilled in the art that, for more details of the venting mechanism 300 in these embodiments, reference is made to the above description in connection with the preceding embodiments, and further description thereof is omitted herein.

According to these alternative embodiments, the venting mechanism 300 may operate with the press button 310 being at the rest position during normal use (when there is no need for venting). Since the venting chamber 120 communicates with the transition chamber 130, air and the liquid can flow freely into the venting chamber 120. Moreover, since the air valve 320 is closed in this configuration, the venting chamber 120 is isolated from the external environment, disallowing release of air into the external environment. When there is a need for venting, the press button 310 may be pressed down (if the locking member 160 is included, it may be displaced outwardly until it is separate from the press button 310, before the press button 310 is pressed down) to urge the bottom end of the venting rod 330 towards the transition chamber 130. As a result, the bottom end gradually moves away from the air valve 320, and once it is completely separate from the air valve 320, air in the venting chamber 120 flows into the mounting chamber 150 through the air valve 320 and further into the outside world from the mounting chamber 150. Thus, venting of air is achieved. Once the press button 310 has been pressed down by a definite distance, its further downward movement will cause the air-valve cap 360, and hence the air valve 320, to move downwards therewith, against the resilient member. Thus, when the press button 310 is released, the resilient member 350 will bias the press button 310 upwards, causing the venting rod 330 and the air-valve cap 360 to move upwards therewith. At this point, since the air valve 320 is spaced from the sealing head 332 of the venting rod 330 at a certain distance, the air valve 320 is not closed immediately. Instead, the air valve 320 is closed at a later time when the sealing head 332 comes into contact with it. As a result of the air valve 320 being closed, the venting chamber 120 is isolated from the external environment. Moreover, at this point, the biasing force of the resilient member 350 still exists and urges the venting rod 330, the air valve 320, the air-valve cap 360 and the press button 310 towards their rest positions. This creates a negative pressure within the venting chamber 120, which in turn creates an effect of sucking the liquid and air away from the balloon. These venting operations may be repeated until air is completely vented (if the locking member 160 is included, after complete air venting is attained, the locking member 160 may be again moved into abutment with the bottom of the press button 310 to bring the venting mechanism 300 into a locked state, before cyclic balloon inflation is initiated).

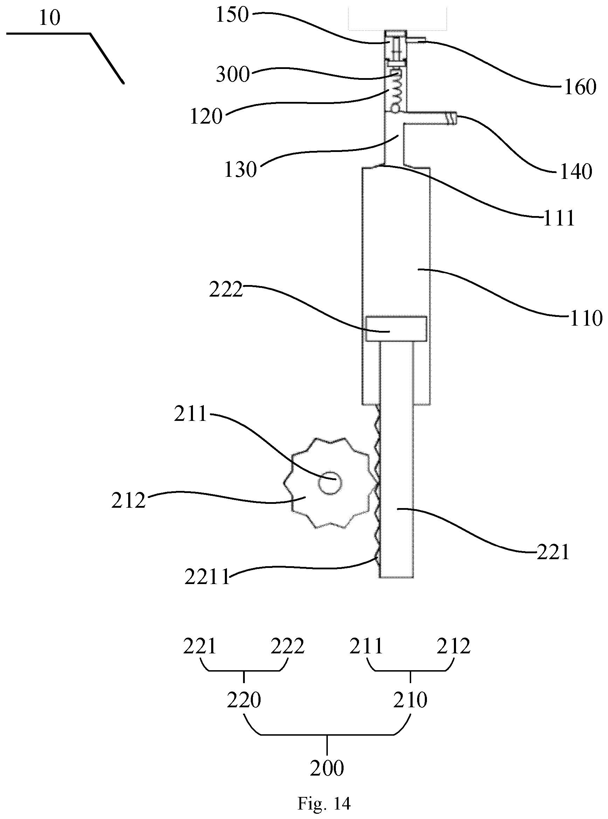

Reference is made further to Fig. 14, a schematic diagram showing the overall structure of a liquid pump 10, according to an alternative embodiment of the present invention. As shown in Fig. 14, the liquid pump 10 of this embodiment differs from that shown in Fig. 1 primarily in that the liquid pump 10 shown in Fig. 1 is of a horizontal structure where the axis of the liquid storage chamber 110 is perpendicular to the axis of the venting chamber 120, while the liquid pump 10 shown in Fig. 14 is of a vertical structure where the axis of the liquid storage chamber 110 is parallel to the axis of the venting chamber 120. It should be noted and will be appreciated by those skilled in the art that, for more structural details of the liquid pump 10 of this embodiment, reference is made to the above description in connection with the structure of the liquid pump 10 of the foregoing embodiments, and further description thereof is omitted herein. Also, it should be noted and will be appreciated by those skilled in the art that the venting mechanism 300 shown in Figs. 12 and 13 is also applicable to the liquid pump 10 of this embodiment.

The above object is also attained by a liquid filling system disclosed herein. Reference is now made to Fig. 15, a schematic block diagram of a liquid filling system, according to an embodiment of the present invention. As shown in Fig. 15, a liquid filling system, as disclosed herein, includes a main unit 20 and a liquid pump 10, as discussed above. The main unit 20 is detachably coupled to the liquid pump 10. Since the liquid filling system, as disclosed herein, incorporates the liquid pump 10, as disclosed herein, it offers at least all the benefits of the liquid pump 10. These benefits are not repeated herein, and reference is made to the above description in connection with the liquid pump 10 for more details of them. Additionally, the liquid pump 10 contains a liquid, which can be communicated into the balloon. In an extreme case, if the balloon ruptures within a human blood vessel, the liquid would leak from the liquid pump 10 and come into direct contact with blood in the human body. Therefore, it would be necessary to sterilize and disinfect the liquid pump 10 each time before clinical use. According to the present invention, since the liquid pump 10 is detachably coupled to the main unit 20 in the liquid filling system, the liquid pump 10 could be sterilized by the manufacturer and then provided in a sterile form. This allows for direct clinical use of a fresh, sterile liquid pump 10 every time by a user, and coupled with the fact that sterile saline or a similar sterile liquid that is readily available in an operating room could be used as the liquid in the liquid pump 10, dispenses with repeatedly sterilization and disinfection of the liquid filling system and medical microcirculation device by hospital personnel, addressing the problem of sterilization and disinfection of the liquid pump 10 and the main unit 20 that would be otherwise necessary every time before clinical use if the liquid pump 10 is integrated with the main unit 20. Further, the design with the liquid pump 10 being detachably coupled to the main unit 20 enables easy and convenient maintenance of the liquid pump 10 and, in the event of the liquid pump 10 being damaged or otherwise rendered inoperable, allows it to be readily exchanged for a new one that could be quickly assembled on the main unit 20.

The liquid filling system, as disclosed herein, may operate with the press button 310 being at the rest position during normal use (when there is no need for venting). Since the venting chamber 120 communicates with the transition chamber 130, air and the liquid can flow freely into the venting chamber 120. Moreover, since the air valve 320 is closed in this configuration, the venting chamber 120 is isolated from the external environment, disallowing release of air into the external environment. When there is a need for venting, the press button 310 may be pressed down (if the locking member 160 is included, it may be displaced outwardly until it is separate from the press button 310, before the press button 310 is pressed down) to urge the bottom end of the venting rod 330 towards the transition chamber 130. As a result, the bottom end gradually moves away from the air valve 320, and once it is completely separate from the air valve 320, air in the venting chamber 120 flows into the mounting chamber 150 through the air valve 320 and further into the outside world from the mounting chamber 150. Thus, venting of air is achieved. Once the press button 310 has been pressed down by a definite distance, its further downward movement will cause the air-valve cap 360, and hence the air valve 320, to move downwards therewith, against the resilient member. Thus, when the press button 310 is released, the resilient member 350 will bias the press button 310 and the venting rod 330 upwards, bringing the air-valve cap 360 away from the air valve 320. At this point, since the air valve 320 is spaced from the sealing head 332 of the venting rod 330 at a certain distance, the air valve 320 is not closed immediately. Instead, the air valve 320 is closed at a later time when the sealing head 332 comes into contact with it. As a result of the air valve 320 being closed, the venting chamber 120 is isolated from the external environment. Moreover, at this point, the biasing force of the resilient member 350 still exists and urges the venting rod 330, the air valve 320, the air-valve cap 360 and the press button 310 towards their rest positions. This creates a negative pressure within the venting chamber 120, which in turn creates an effect of sucking the liquid and air away from the balloon. These venting operations may be repeated until air is completely vented (if the locking member 160 is included, after complete air venting is attained, the locking member 160 may be again moved into abutment with the bottom of the press button 310 to bring the venting mechanism 300 into a locked state, before cyclic balloon inflation is initiated). Thereafter, the piston 220 in the inflation/deflation mechanism 200 may be driven to move along the axis of the liquid storage chamber 110 toward the balloon to transfer the inflating liquid from the liquid storage chamber 110 into the balloon to cause its inflation. In order to deflate the balloon, the piston 220 in the inflation/deflation mechanism 200 may be driven to move along the axis of the liquid storage chamber 110 away from the balloon to draw the liquid back into the liquid storage chamber 110 from the balloon. It should be noted and will be appreciated by those skilled in the art that, prior to air venting, the connector 140 on the pump body 100 is connected to a liquid reservoir that contains the inflating liquid, and the inflation/deflation mechanism 200 is then activated to draw the inflating liquid into the liquid storage chamber 110 from the liquid reservoir.

It should be noted and will be appreciated by those skilled in the art that the main unit 20 may include, but is not limited to including, a controller, a display screen and a power device 21 (e.g., an electric motor) as discussed above. For more structural details of the main unit 20, reference is made to literature on related techniques known to those skilled in the art, and further description thereof is omitted herein.

The above object is also attained by a medical microcirculation device disclosed herein. Reference is now made to Fig. 16, a schematic block diagram of a medical microcirculation device, according to an embodiment of the present invention. As shown in Fig. 16, a medical microcirculation device, as disclosed herein, includes a balloon catheter 30 and a liquid filling system, as discussed above. Since the medical microcirculation device, as disclosed herein, incorporates the liquid filling system, as disclosed herein, it offers at least all the benefits of the liquid filling system. These benefits are not repeated herein, and reference is made to the above description in connection with the liquid filling system for more details of them.

It should be noted that the present invention is not limited to any particular structure of the balloon catheter 30. For more structural details of the balloon catheter 30, reference is made to literature on related techniques known to those skilled in the art, and further description thereof is omitted herein. Also, it should be noted and will be appreciated by those skilled in the art that, in addition to the balloon catheter 30 and the liquid filling system discussed above, the medical microcirculation device, as disclosed herein, may also include other components. For more structural details of the medical microcirculation device, reference is made to literature on related techniques known to those skilled in the art, and further description thereof is omitted herein. Further, it should be noted and will be appreciated by those skilled in the art that the medical microcirculation device, as disclosed herein, can be used to treat not only obstructive coronary disease in the microcirculatory system around the coronary sinus, but also other obstructive arterial diseases.

In summary, the liquid pump 10, the liquid filling system and the medical microcirculation device, as disclosed herein, offer the following benefits over the prior art:
The liquid storage chamber 110 and the inflation/deflation mechanism 200, as disclosed herein, allow for balloon inflation by liquid filling, reducing the risk of gas embolism caused by gas leakage during use. Moreover, the transition chamber 130, the venting chamber 120 and the venting mechanism 300, as disclosed herein, overcome the problem of complex venting operations, while providing satisfactory venting during liquid filling. In addition, the design with the liquid pump 10 being detachably coupled to the main unit 20, as disclosed herein allows the liquid pump 10 to be sterilized by the manufacturer and then provided in a sterile form. This enables direct clinical use of a fresh, sterile liquid pump 10 every time by a user, and coupled with the fact that sterile saline or a similar sterile liquid that is readily available in an operating room could be used as the liquid in the liquid pump 10, dispenses with repeatedly sterilization and disinfection of the liquid filling system and medical microcirculation device by hospital personnel, addressing the problem of sterilization and disinfection of the liquid pump 10 and the main unit 20 that would be otherwise necessary every time before clinical use if the liquid pump 10 is integrated with the main unit 20. This design also enables easy and convenient maintenance of the liquid pump 10 and, in the event of the liquid pump 10 being damaged or otherwise rendered inoperable, allows it to be readily exchanged for a new one that could be quickly assembled on the main unit 20.

The description presented above is merely that of a few preferred embodiments of the present invention and is not intended to limit the scope thereof in any sense. Any and all changes and modifications made by those of ordinary skill in the art in light of the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A liquid pump for use in a medical microcirculation device, comprising a pump body, an inflation/deflation mechanism and a venting mechanism,
the pump body comprising a liquid storage chamber, a venting chamber and a transition chamber joining the liquid storage chamber to the venting chamber, the transition chamber disposed above the liquid storage chamber and communicating with the liquid storage chamber,
the pump body further comprising a connector adapted for connection with a balloon catheter, and the connector communicating with the transition chamber,
at least a portion of the inflation/deflation mechanism disposed within the liquid storage chamber and being able to reciprocate along an axis of the liquid storage chamber to inflate and/or deflate a balloon,
at least a portion of the venting mechanism disposed within the venting chamber and being able to reciprocate along an axis of the venting chamber, thereby bringing the venting chamber into communication with, or isolating the venting chamber from, an external environment.

2. The liquid pump according to claim 1, wherein the inflation/deflation mechanism comprises a transmission assembly and a piston, which are connected to each other, the piston comprising a piston rod and a piston head, which are connected to each other, the piston head disposed within the liquid storage chamber, the piston extending, at least in part, outside the liquid storage chamber and drivingly connected to the transmission assembly, the transmission assembly connected to a power device and configured to be driven by the power device to cause the piston rod and the piston head to reciprocate along the axis of the liquid storage chamber.

3. The liquid pump according to claim 2, wherein the transmission assembly comprises a coupling and a gear coaxially connected to the coupling, wherein the piston rod is drivingly connected to the gear, and wherein the coupling is connected to the power device.

4. The liquid pump according to claim 3, wherein there is a plurality of teeth on one side of the piston rod, which are configured to mesh with the gear.

5. The liquid pump according to claim 3, wherein the piston rod is eccentrically connected to the gear via a joint member.

6. The liquid pump according to claim 5, wherein the joint member is eccentrically affixed to the gear at one end and articulated to the piston rod at the other end, and wherein the piston rod is articulated to the piston head.

7. The liquid pump according to claim 1, wherein the liquid storage chamber comprises a tapered portion at an end thereof proximal to the transition chamber, and wherein an inner diameter of the tapered portion at its end proximal to the transition chamber less than an inner diameter of the tapered portion at its end distal from the transition chamber.

8. The liquid pump according to claim 1, wherein the venting mechanism comprises a press button, an air valve and a venting rod, a top end of the venting rod connected to the press button, the air valve movably sleeved on the venting rod, wherein: the pump body further comprises a mounting chamber joined to the venting chamber, the mounting chamber communicating with the external environment; top ends of the press button and the venting rod are both disposed within the mounting chamber; the press button is able to reciprocate along an axis of the mounting chamber; a bottom end of the venting rod is disposed in the venting chamber; the air valve is located at a joint of the mounting chamber and the venting chamber; when the press button is at rest, the bottom end of the venting rod seals a bottom end of the air valve, thereby isolating the venting chamber from the mounting chamber; when the press button is pressed, the venting rod is caused to move toward the transition chamber relative to the air valve, thereby bringing the venting chamber into communication with the mounting chamber.

9. The liquid pump according to claim 8, further comprising a locking member movably connected to a wall of the mounting chamber, the locking member arranged below the press button and above the air valve, the locking member extending, at least in part, outside the mounting chamber, the locking member configured to, when there is no need for venting, abut against the press button, thereby placing the venting mechanism into a locked state.

10. The liquid pump according to claim 8, wherein the venting rod comprises a rod body and a sealing head, which are connected to each other, the sealing head having a diameter greater than a diameter of the rod body, a top end of the rod body connected to the press button, wherein the air valve defines a first bore and a second bore communicating with the first bore, the second bore underlying the first bore, the second bore having a greater inner diameter than the first bore, the second bore configured for insertion of the sealing head therein with an interference fit, the first bore configured for insertion of the rod body therethrough.

11. The liquid pump according to claim 10, wherein the venting mechanism further comprises an air-valve cap disposed within the mounting chamber, the air-valve cap sleeving on the venting rod, the air-valve cap arranged above the air valve, the air-valve cap having a diameter greater than the inner diameter of the first bore, wherein when the press button is at rest, the air-valve cap is spaced from the air valve at a distance greater than a depth of the second bore.

12. The liquid pump according to claim 8, wherein the venting mechanism further comprises a resilient member connected to the press button or the venting rod, the resilient member configured to, when an external force exerted on the press button is removed, bias the venting rod and the press button back to their own rest positions.

13. The liquid pump according to claim 8, wherein the venting mechanism further comprises a ball valve connected to the bottom end of the venting rod, and wherein the venting chamber defines, at an end thereof proximal to the transition chamber, an air inlet port, in which the ball valve is able to be fitted.

14. The liquid pump according to claim 8, wherein the mounting chamber is provided therein with a stop member, the stop member arranged between the press button and the air valve.

15. A liquid filling system, comprising a main unit and the liquid pump as defined in any one of claims 1 to 14, the main unit detachably connected to the liquid pump.

16. A medical microcirculation device, comprising a balloon catheter and the liquid filling system as defined in claim 15.
